# EUROPEAN PATENT APPLICATION

(11) **EP 1 236 474 A1**
(43) Date of publication of application: **04.09.2002**
(21) Application number: 01104737.0
(22) Date of filing: 26.02.2001
(51) Int. Cl.: A61K 38/17, A61K 31/70, A61P 35/00

(54) **Negative regulation of epidermal growth factor receptor activity by Mig-6**

(71) Applicant: Max-Planck-Gesellschaft zur Förderung der Wissenschaften e.V., 80539 München (DE)
(72) Inventor: Ullrich, Axel, Dr., 80799 München (DE); Hackel, Peter, 8304 Wallisellen (CH)
(74) Representative: Weiss, Wolfgang, Dr. Dipl.-Chem.

(57) **Abstract**

The present invention relates to the use of a Mig-6 protein or a nucleic acid coding therefor for the manufacture of an agent for the modulation of epidermal growth factor receptor (EGFR) activity. Further, the use of Mig-6 or nucleic acids coding therefor as a target for the modulation of EGFR receptor activity is disclosed. The invention also relates to a method for identifying novel substances capable of modulating EGFR activity. The modulation of EGFR activity preferably comprises an inhibition of the EGFR signal and is thus suitable for applications, particularly diagnostic or medical applications, wherein an inhibition of EGFR activity is desired. Thus, the present invention relates to novel methods for diagnosing, treating or preventing EGFR overexpression associated disorders such as tumors.

## Description

The present invention relates to the use of a Mig-6 protein or a nucleic acid coding therefor for the manufacture of an agent for the modulation of epidermal growth factor receptor (EGFR) activity. Further, the use of Mig-6 or nucleic acids coding therefor as a target for the modulation of EGFR receptor activity is disclosed. The invention also relates to a method for identifying novel substances capable of modulating EGFR activity. The modulation of EGFR activity preferably comprises an inhibition of the EGFR signal and is thus suitable for applications, particularly diagnostic or medical applications, wherein an inhibition of EGFR activity is desired. Thus, the present invention relates to novel methods for diagnosing, treating or preventing EGFR overexpression associated disorders such as tumors.

In multicellular organisms, communication between individual cells is essential for the regulation of complex biological processes such as growth, differentiation, motility or survival. In the cellular context, receptor tyrosine kinases (RTKs) are primary mediators of signals from the cell surface to target proteins in cytoplasmic compartments and the nucleus. Within the large family of RTKs the epidermal growth factor receptor (EGFR) subfamily plays a particularly important and complex role in the development and mature state of higher eukaryotes and in a wide variety of pathological disorders such as cancer. The EGFR subfamily consists of four closely related receptors: the EGFR (also known as ErbB1), HER2 (or ErbB2/neu) for which no ligand has been described so far, HER3 (or ErbB3), which is characterized by an impaired kinase function and HER4 (or ErbB4) (Riese II and Stern, 1998). Phosphorylation of specific receptor tyrosine residues upon ligand stimulation creates binding sites for a defined set of primary signal transducers. Some of these molecules are enzymes, which become tyrosine phosphorylated and activated, such as Src, phospholipidase Cγ (PLCγ) or phosphatidylinositol 3-kinase (P13K). In addition, adapter molecules such as Shc, Grb2, Grb7 or Nck can bind to activated receptors and initate signalling cascades by recruiting additional signal transducing proteins (Hackel et al., 1999; Zwick et al., 1999).

While tremendous progress has been made in understanding the activation mechanisms of these pathways, their attenuation and negative regulation at the receptor level is still poorly understood. At the same time it has been recognized in recent years that negative control of receptor signals and their duration are critical for the definition of the cellular response and that disturbances of these negative regulatory mechanisms lead to pathophysiological consequences. A prime example are retroviral oncogenes such as *v*-erb, *v*-fms, *v*-ras, *v*-raf and *v*-fos whose products all represent structurally altered elements of RTK signal cascades which by various means escape negative control and thereby transmit constitutive signals that cause oncogenic transformation of the cell.

Among the inhibitors of phosphotyrosine-mediated signals, protein tyrosine phosphatases (PTP) are obvious candidates to be negative regulators of tyrosine phosphorylated protein kinases. Although interactions between receptor kinases and phosphatases have been demonstrated (Lammers et al., 1995, Liu and Chernoff, 1997), the specific physiological relevance of these interactions still remains unclear. However, several other inhibitory proteins have been recently characterized. Among these, the transmembrane protein SIRPα1 which was identified as an SHP2 phosphatase substrate has been shown to be a negative regulator of cellular responses that are induced by receptor tyrosine kinases, such as the EGFR or the platelet derived growth factor (PDGFR) and a possible role of tumor suppressor has been suggested (Kharitonenkov et al., 1997). Another negative regulator of EGFR signalling has been identified through genetic studies in C. elegans. Sli-1, the invertebrate homologue of the mammalian proto-oncogene c-Cbl, was shown to regulate vulval induction which is mediated by the C. elegans homologue of EGFR (Yoon et al., 1995). Recently, it was shown that c-Cbl regulates endocytic sorting of the EGFR and functions as a ubiquitin-protein ligase and thereby is involved in RTK downregulation and targeting to proteosome degradation (Levkowitz et al., 1998; Joazeiro et al., 1999; Watermann et al., 1999). Interestingly, in Drosophila, kek1, a single pass transmembrane protein with features of a cell adhesion molecule, acts in a negative feedback loop that modulates EGFR activity (Ghiglione et al, 1999).

Downregulation, another attenuation mechanism for surface receptors, is a complex process that involves internalization and degradation as well as recycling pathways. Upon binding of EGF to its surface receptor the autophosphorylated receptor gets clustered into coated pits and is rapidly internalized (Sorkin and Waters, 1993a). This ligand-dependent internalization appears to be a characteristic of the EGFR and has so far not been described for other EGFR-family members (Baulida et al., 1996). The clathrin adaptor AP2 complex, an important structural component of coated pits, has been shown to bind the EGFR in vivo in an EGFR dependent fashion (Sorkin and Carpenter, 1993b). Interestingly, however, a mutant EGFR lacking the AP2 binding determinant shows internalization and downregulation kinetics indistinguishable from the wildtype receptor (Nesterov et al., 1995). This result indicates that processes other than adaptor-mediated retentation in coated pits are required for EGFR endocytosis. Moreover, several additional sequences and motifs in the carboxy-terminal domain of the EGFR were described to be involved in the internalization and/or degradation process (Sorkin et al., 1992; Chang et al., 1993; Kornovila et al., 1996). Kinase activity as well as downstream signalling and activation of as yet unidentified substrates appear to be necessary for this ligand induced endocytosis (Honegger et al., 1987; Lamaze and Schmid, 1995). After internalization, growth factor receptors get translocated into internal vesicles of the multivesicular body and segregated either for recycling or lysosomal/proteosomal degradation. This process of segregation is also involved in attenuation of RTK signalling and several molecules, such as annexin I (Futter et al., 1993), SNX1 (Kurten et al., 1996), PI3K (Joly et al., 1995); Grb2 (Wang and Moran, 1996) or c-Cbl (Levkowitz et al., 1998), have been suggested to be playing a role in it.

Desensitization of the EGFR may also involve the phosphorylation of receptor serine and threonine residues. Four major serine phosphorylation sites on the EGFR have been mapped; serine S671, S1002, S1046 and S1047 (Heisermann and Gill; 1988; Kuppuswamy et al., 1993). A role in the process of EGFR desensitization has been suggested for S1002 (Kuppuswamy et al., 1993) as well as for SS1046/1047 (Counterway et al., 1992; Theroux et al., 1992a; Theroux et al., 1992b). In addition, it has been shown that the protein serine/theronine kinase C (PKC) downmodulates the signalling potential of the EGFR by an MAPK/ERK kinase (MEK) dependent mechansim (Morrison et al., 1996) and that EGFR kinase activity is decreased by prolactin-induced threonine phosphorylation of the receptor (Quijano Jr. and Sheffield, 1998).

Besides the role of the EGF-signalling in normal mitogenesis, aberrant expression or structural alterations of the receptor or its ligand are involved in oncogenesis. The EGFR is amplified in human cancers includding bladder (Proctor et al., 1991), breast (Horak et al., 1991) and colon tumors (Radinsky et al., 1995) and different truncation mutants of the EGFR have been detected in glioblastoma and carcinomas of the lung, breast and ovary (Hesketh, 1997). In addition, the impairment of EGFR negative regulation has been connected to cell transformation and tumorigenicity (Wells et al., 1990; Ekstrand et al., 1992). In different *v*-erbB oncogenes, for example, the negative regulating serine phosphorylation sites SS1046/1047 are consistently found to be deleted and this mutation correlates with increased oncogenic potential (Theroux et al., 1992a). These examples indicate that comprehensive understanding of the mechanisms that negatively regulate mechanisms that negatively regulate RTK signalling will provide new insights into the process of oncogenesis and may yield novel strategies for cancer therapy.

In present study, we used a yeast two-hybrid approach to identify new interaction partners of the EGFR, which are involved in the negative regulation of its signal capacity. Among well known as well as unknown interaction partners, Mig-6 was identified. Mig-6 interacts with the EGFR in vivo in an EGF inducible manner. Overexpression in Cos-7 cells as well as in Rat-1 fibroblasts leads to accelerated dephosphorylation of the activated receptor. Mig-6 reduces MAPK activation upon EGF stimulation and suppresses focus formation induced by overexpression of the EGFR. Moreover, the transcription of Mig-6 mRNA is induced upon EGF stimulation which suggest a role of Mig-6 in negative feedback regulation of the EGFR signal.

A human Mig-6 gene was cloned by Wick et al. (1995). Recently Fiorentino et al. (2000) have described an inhibition of ErbB-2 mitogenic and transforming activity by RALT, a Mig-6 analog from rat, which binds to the ErbB-2 kinase domain. An interaction between Mig-6 and EGFR is, however, neither disclosed nor suggested.

Thus, a first aspect of the present invention relates to the use of a Mig-6 protein for the manufacture of an agent for the modulation, particularly for the inhibition of EGFR activity.

A further aspect of the present invention relates to the use of a nucleic acid encoding a Mig-6 protein or a nucleic acid complementary thereto for the manufacture of an agent for the modulation, particularly for the inhibition of EGFR activity.

A third aspect of the present invention relates to the use of a Mig-6 protein as a target for the modulation of EGFR activity.

A fourth aspect of the present invention relates to the use of a nucleic acid encoding a Mig-6 protein or a nucleic acid complementary thereto as a target for the modulation of EGFR activity.

A fifth aspect of the present invention relates to a method for identifying novel modulators of EGFR activity by screening for substances having an equivalent biological activity of Mig-6.

The term "Mig-6 protein" as used in the present application particularly encompasses mammalian Mig-6 proteins, such as Mig-6 proteins from man, mouse, rat, hamster, monkey, pig, etc. The sequence of several mammalian Mig-6 DNAs and the corresponding proteins are disclosed in Genbank Accession No. M23572 (rat), NM_018948 (human) and AK004851 (mouse) and citations therein, incorporated herein by reference.

Especially preferred is a human Mig-6 protein comprising:
(a) the amino acid sequence as shown in Genbank Accession No. NM_018948 or
(b) an amino acid sequence having an identity of at least 80%, particularly of at least 90% and more particularly of least 95% thereto, wherein the amino acid sequence identity may be determined by a suitable computer program such as GCG.

Furthermore, the term "Mig-6 protein" encompasses recombinant derivatives or variants thereof as well as fragments capable of binding to EGFR and preferably thereby inhibiting EGFR signal activity. Such derivatives, variants and fragments are obtainable by recombinant expression of corresponding nucleic acids in a suitable host cell and obtaining the resulting expression products by known methods. The activity of the resulting expression products may be determined according to the methods described in the present application, particularly in the examples section.

The Mig-6 protein is encoded by a nucleic acid, which may be a DNA or an RNA. Preferably, the nucleic acid comprises:
(a) the nucleic acid sequence as shown in Genbank Accession No. NM_018948 or complementary thereto,
(b) a nucleic acid sequence corresponding to the sequence of (a) within the scope of degeneracy of the genetic code or
(c) a nucleic acid sequence hybridizing under stringent conditions with the sequence of (a) and/or (b).

The term "hybridization under stringent conditions" according to the present application is used as described in Sambrook et al., Molecular Cloning, A Laboratory Manual, Cold Spring Harbor, Laboratory Press (1989), 1.101-1.104. Consequently, hybridization under stringent conditions occurs when a positive hybridization signal is still detected after washing for 1 h with 1 x SSC and 0.1% SDS at 55°C, preferably at 62°C and most preferably 68°C, in particular for 1 h in 0.2 x SSC and 0.1% SDS at 55°C, preferably at 62°C and most preferably at 68°C. A nucleotide sequence hybridizing under such washing conditions with a sequence as shown in the sequence listing or a complementary nucleotide sequence or a sequence within the scope of degeneracy of the genetic code is encompassed by the present invention.

The nucleic acid molecules of the invention may be recombinant nucleic acid molecules generated e.g. by known amplification methods e.g. PCR. On the other hand, the nucleic acid molecules can also be chemically synthesized nucleic acids. Preferably, the nucleic acid molecules are present in a vector, which may be any prokaryotic or eukaryotic vector, on which the nucleic acid sequence is present preferably under control of a suitable expression signal, e.g. promoter, operator, enhancer etc. Examples for prokaryotic vectors are chromosomal vectors such as bacteriophages and extrachromosomal vectors such as plasmids, wherein circular plasmid vectors are preferred. Examples for eukaryotic vectors are yeast vectors or vectors suitable for higher cells, e.g. insect cells or mammalian cells, plasmids or viruses.

The Mig-6 protein is capable of binding to a member of the EGF receptor subfamily, particularly EGFR and thereby inhibiting the receptor activity, particularly the receptor signal. This inhibition in turn may lead to an inhibition of the mitogen-activated protein kinase pathway, particularly, an inhibition of the protein kinase Erk2 activity. Thus, a Mig-6 protein of the present invention preferably binds to EGFR in a region between amino acids 985 and 995 (nomenclature?), wherein said binding is preferably independent of tyrosine 992. This binding results in a selective inhibition of the mitogen-activated protein kinase pathway in response to EGF, wherein substantially no inhibition is found in response to other growth factors such as FGF (fibroblast growth factor) or PDGF (platelet derived growth factor). Furthermore, administration of Mig-6 may result in an enhanced receptor internalization without substantially effecting the rate of degradation.

Due to this biological activity, Mig-6 is a suitable agent for the diagnosis, prevention or treatment of an EGFR overexpression associated disorder. This disorder may be a hyperproliferative disease which may be selected from inflammatory processes and tumors such as cancers of bladder, breast and colon and gliobastomas or carcinomas of lung, breast and ovary.

The Mig-6 protein may be delivered to a target cell in any form which may interact with the receptor. For example Mig-6 may be administered directly into a cell by using suitable vehicles capable of being internalized into the target cell such as liposomes. These vehicles may comprise a target specific molecule, particularly a molecule which is specific for tumor cells. Alternatively, Mig-6 may be administered in form of its nucleic acid by means of a gene therapeutic method, preferably by using viral vectors such as adenoviruses, retroviruses or adeno-associated viruses.

The administration of the Mig-6 protein or the nucleic acid encoding a Mig-6 protein or a nucleic acid complementary thereto is preferably in form of a pharmaceutical composition which additionally comprises suitable pharmaceutically acceptable carriers or diluents. The composition may be an injectable solution, a suspension, a cream, an ointment, a tablet etc. This composition is suitable for diagnostic or medical, e.g. preventive or therapeutic applications particularly in the field of cancer. The dosage and administration route depends on the type and severity of the disorder to be treated and may be determined readily by a skilled practician.

The administration of the protein may be carried out according to known protocols. The administration in form of the nucleic acid may also be carried out in form of known protocols. The administration of Mig-6 proteins or nucleic acids may be combined with the administration of other active agents, particularly anti-tumor agents, e.g. cytotoxic substances and EGFR inhibitors such as metalloproteinase inhibitors like marimastat or batimastat.

Furthermore, the Mig-6 protein is a suitable target for the modulation of EGFR activity. In this embodiment of the present invention an effector of the Mig-6 protein is administered to a cell or an organism having an EGFR associated disorder. The effector is preferably a substance which is capable of enhancing the amount and/or activity of a Mig-6 protein in the target cell or target organism. For example, the effector may be capable of enhancing the expression of Mig-6 in a target cell, e.g. by activating transcription or translation of a native Mig-6 gene in this target cell.

Furthermore, an activator of Mig-6, e.g. a substance which enhances the biological activity of Mig-6, may be administered to the target cell.

Still a further embodiment of the present invention is a method of identifying novel modulators of EGFR activity comprising screening for substances having equivalent biological activity as Mig-6. In this context, the term "biological activity of Mig-6" preferably comprises binding to EGFR in a region between amino acids 985 and 995, wherein said binding is more preferably independent of tyrosine 992 and results in a selective inhibition of the mitogen-activated protein kinase in response to EGF.

The screening method may be a high-throughput screening method, wherein a plurality of substances is tested in parallel. The screening assay may be a cellular assay or a molecular assay, wherein an interaction of a substance to be tested with the Mig-6 binding region of EGFR is determined. The EGFR may be provided on a cell, preferably an EGFR overexpressing cell, an EGFR containing cell fraction or a substantially isolated and purified EGFR or a fragment thereof which is capable of binding Mig-6. Any active substance identified by this method may be used as a pharmaceutical agent or as a lead structure which is further modified to improve pharmaceutical properties.

The present invention is explained in more detail in the following figures and examples.

### Figure description

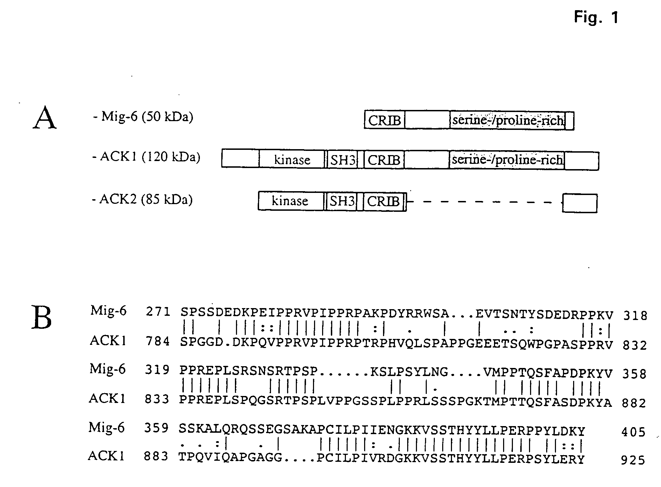

- Fig. 1: A) Mig-6 homology comparison.
   Mig-6, ACK1 and ACK2 contain a Cdc-42 and Rac interaction binding (CRIB) motif. Mig-6 and ACK1 share sequence homology within a carboxy-terminal serine- and proline-rich region, which is deleted in ACK2. In addition, ACK1 and ACK2 contain a kinase and an SH3 domain.
B) Amino acid homology of 71% in the serine- and proline-rich carboxy terminal domain between Mig-6 and ACK1 (shaded region in A). The amino acid sequences were aligned with the GCG computer program.

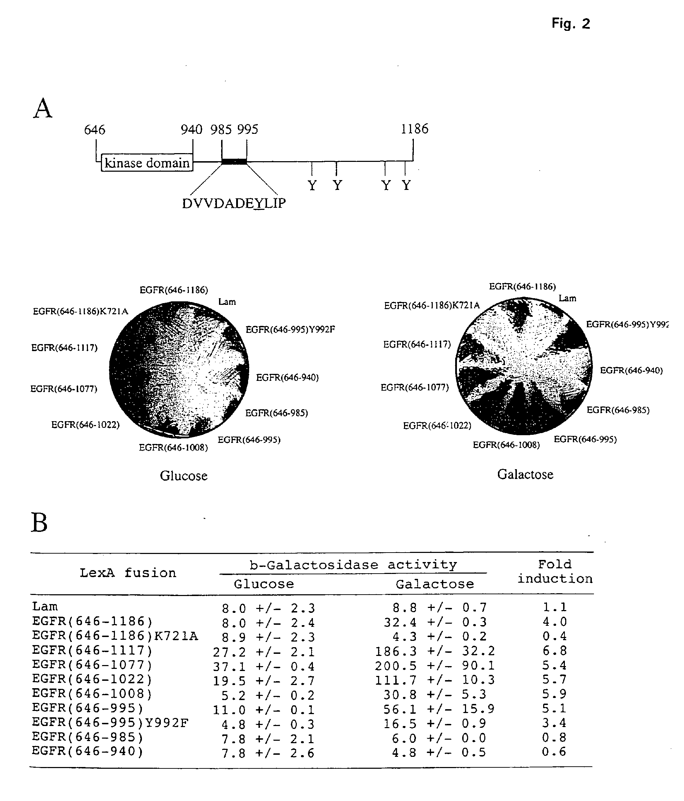

- Fig. 2: Interaction between Mig-6 and the EGFR in the yeast two-hybrid system.
A) Representative examples of colonly growth. Laminin, the intracellular domain of the EGFR, the kinase impaired EGFR mutant K721A or the indicated truncations with full length Mig-6. The strains were streaked on synthetic media containing glucose or galactose and lacking leucine, tryptophan, histidine and uracil.
B) β-galactosidase activity assays. Strains containing the same constructs as in A) were grown in liquid synthetic media containing glucose or galactose and lacking tryptophan, histidine and uracil. o-Nitrophenyl β-D-galactopyranoside was used as a substrate and β-galactosidase activity was calculated from absorbance measurements (OD) at 420 nm and 600 nm and expressed as OD₄₂₀/OD₆₀₀*1000. The data from three independent experiments and the standard deviation are indicated.

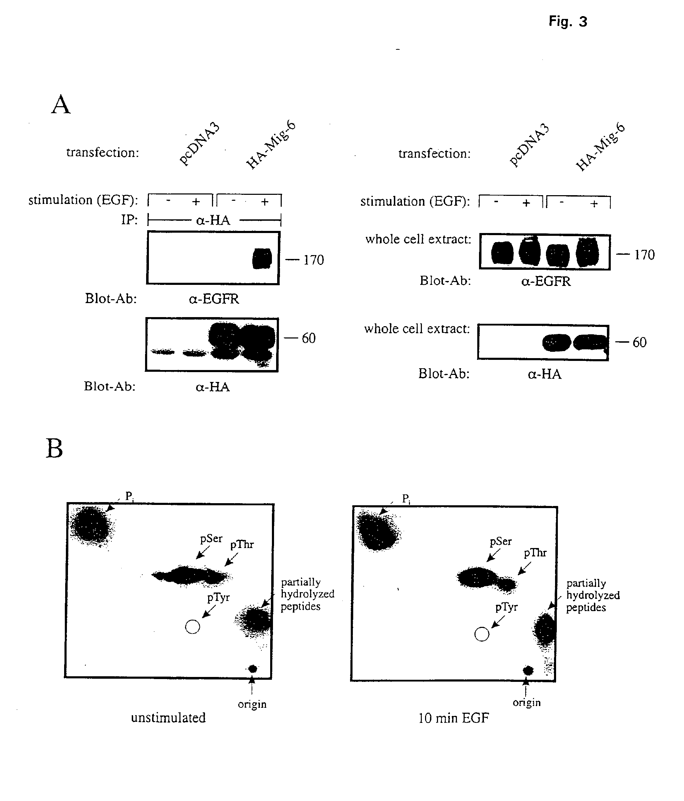

- Fig. 3: Co-immunoprecipitation of the endogenous EGFR with HA-Mig-6 and phosphoamino acid analysis of Mig-6.
A) Cos-7 cells were transiently transfected with cDNA encoding HA-Mig-6 or control-transfected with the empty vector. After starvation, the cells were stimulated with 10 ng EGF/ml for 10 min, lysed and HA-Mig-6 was immunoprecipitated with a monoclonal anti-HA antibody. After gel electrophoresis, the precipitated HA-Mig-6 was detected by immunoblotting with anti-HA antibody and the coprecipitated EGFR was detected with an anti-EGFR antibody. Expression of transfected HA-Mig-6 and endogenous EGFR was checked by probing the whole cell extract with the respective antibodies.
B) For phosphoamino acid analysis of Mig-6, Cos-7 cells were transfected with cDNA encoding Mig-6, starved and stimulated with 10 ng EGF/ml for different periods of time between 10 and 60 min (representive data of unstimulated and after 10 min stimulation are shown). After immunoprecipitation, the proteins were gel eletrophoresed, transferred to a PVDF membrane and subjected to acid hydrolysis. The amino acids were then separated by two-dimensional thin layer electrophoresis on cellulose plates and detected by autoradiography. Exposure was at -80°C using an intensifying screen.

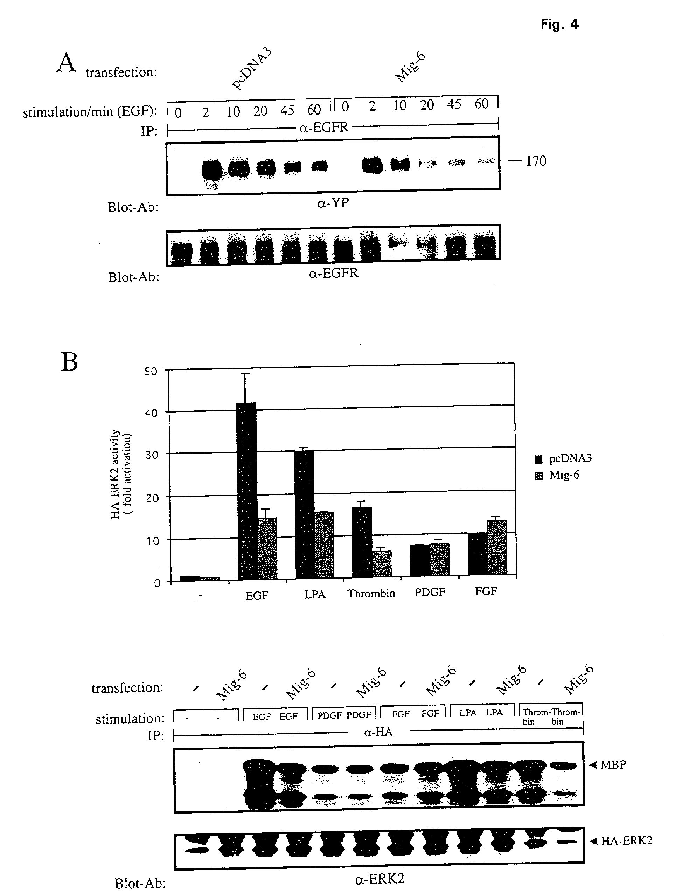

- Fig. 4: Mig-6 accelerates EGFR dephosphorylation and reduces MAPK activation upon EGF stimulation.
A) Cos-7 cells were transiently transfected with cDNA encoding Mig-6 or control-transfected with the empty vector. Quiescent cells were stimulated with 10 ng EGF/ml as indicated. After lysis the EGFR was precipitated with a monoclonal anti-EGFR and subsequently immunoblotted with a monoclonal anti-phosphotyrosine (α-PY) antibody. The amount of EGFR in immunoprecipitates was determined by reprobing the same membrane with an anti-EGFR antibody (lower panel).
B) Cos-7 cells were transiently cotransfected with an expression plasmid encoding HA-Erk2 (100 ng/well) and a plasmid encoding Mig-6 (200 ng/well) or the empty vector. Following serum starvation for 24 hours, cells were stimulated for 7 min with 10 ng/EGF, 10 µM LPA, 2 U/ml thrombin, 50 ng PDGF/ml or 30 ng FGF/ml. After lysis and immunoprecipitation of HA-Erk2 with a monoclonal anti-HA antibody, HA-Erk2 activity was determined using MBP as substrate in an in vitro kinase assay as described in Material and Methods. After gel electrophoresis, phosphorylated MBP was visualized by autoradiography and quantified using a Phosphoimager (upper panel). The amount of precipitated HA-Erk2 was determined by immunoblotting using polyclonal anti-Erk2 antibody (lower panel). The presented data are mean values of two independent experiments.

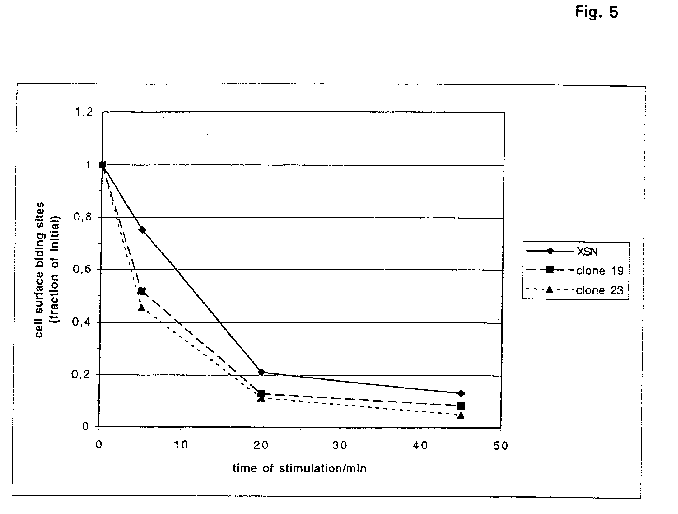

- Fig. 5: Mig-6 downregulates the EGFR.
Two monoclonal Rat-1 cell lines stably expressing Mig-6 (clone 19 and 23) and control cells were superinfected with supernatants of GP+E 86 cells releasing replication defective ectopic pLXSN-EGFR virus. After starvation, the cells were stimulated with 10 ng/ml EGF for the indicated times. The medium was aspirated, the cells were washed and the surface-bound ligand was removed by acid-stripping. The number of ligand-binding sites on the cell surface was then determined by incubating cells with ¹²⁵I-EGF at 4°C for 2 hours. After washing, the surface bound radioactivity was removed by acid stripping and counted with a Beckmann 4000 counter. Representative data from three independent experiments are shown.

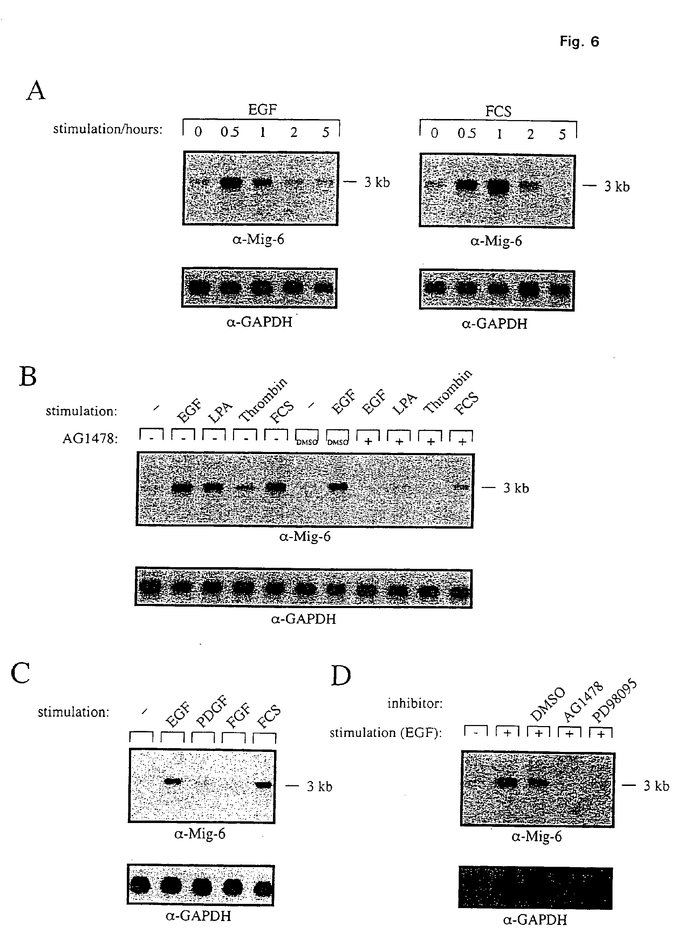

- Fig. 6: Northern blot analysis of Mig-6 induction in Rat-1 fibroblasts.
Total RNA was extracted after the indicated treatment and 10 µg were resolved on a 1.4% agarose-formaldehyde gel. The fractionated RNA was transferred to nitrocellulose membranes for hybridization with a ³²P-labeled cDNA probe of the Mig-6 full length coding sequence. A GAPDH-probe was used to control equal loading and integrity of the RNA (lower panels). Blots were exposed at -80°C using an intensifying screen.
A) mRNA of Mig-6 is transiently expressed upon EGF stimulation. Quiscent cells were stimulated with 50 ng/ml EGF for the indicated time.
B) mRNA expression of Mig-6 is induced upon GPCR agonist-induced EGFR transactivation. Following pre-incubation with or without 100 nM AG1478 for 15 min, quiscent cells were stimulated for 45 min with 20 ng/EGF, 10 mM LPA, 2 U/ml thrombin or 10% FCS.
C) PDGF or FGF stimulation does not induce Mig-6 mRNA expression. Starved cells were stimulated for 45 min with 20 ng/ml EGF, 50 ng/ml PDGF, 30 ng/ml FGF or 10% FCS.
D) Activation of MEK is necessary for induction of Mig-6 mRNA expression. After pre-incubation with or without DMSO, 100 nM AG1478 or 50 µM PD98095, starved cells were stimulated for 45 min with 20 ng/ml EGF.

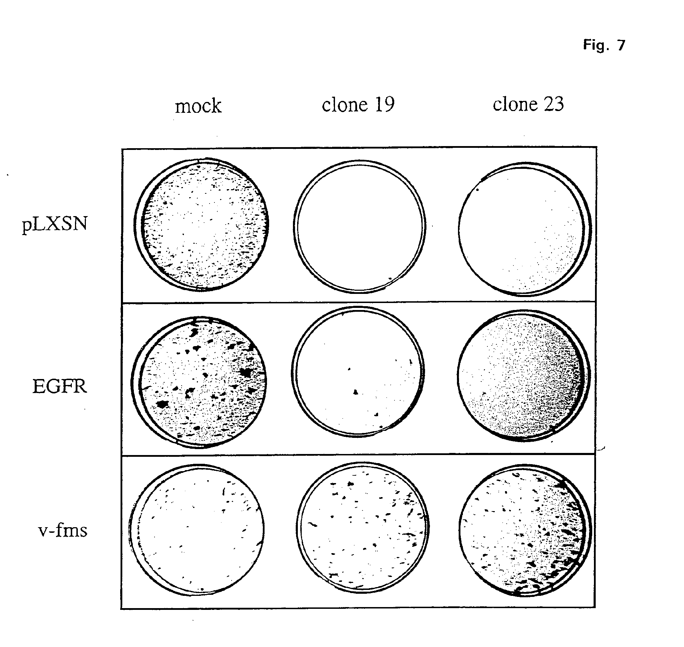

- Fig. 7: EGFR-dependent Rat-1 fibroblast transformation.
Two monoclonal Rat-1 cell lines (10⁵ cells/6 cm dish) that stably express Mig-6 (clone 19 and 23) and control cells (mock) were superinfected with equal amounts (m.o.i. = 0.1-0.25) of pLXSN containing the cDNAs of wildtype EGFR, *v*-fms or no insert. Cells were cultured in the presence of 4% FCS with (EGFR, no insert) or without (*v*-fms) the addition of 10 ng/ml EGF. Medium was changed every other day. After 14 days cell foci were fixed with paraformaldehyde and stained with crystal violet.

### Examples

### 1. Materials and Methods

### 1.1 Reagents, antibodies, plasmids

Culture media and Lipofectamine were purchased from Gibco-BRL, protein A-Sepharose was from Amersham Pharmacia Biotech Inc., AG1478 from Alexis and PD98059 from Calbiochem-Novabiochem. All other reagents were obtained from Sigma. Monoclonal anti-HA antibody was purchased from Roche Diagnostics, rabbit polyclonal anti-EGFR antibody from Santa Cruz, sheep polyclonal anti-EGFR antibody from UBI, monoclonal anti-phosphotyrosine antibody from UBI. For polyclonal anti-Mig-6 antibody, amino acid 271-461 of a Mig-6 clone identified in the yeast two-hybrid screen were expressed as a pGEX fusion protein in Escherichia coli, purified with GSH-Sepharose and used as antigen in rabbits. EGFR from Cos-7 cells was precipitated with mouse monoclonal antibody 108.1 (Seedorf et al., 194). Respective secondary antibodies were obtained from Bio-Rad and Dianova. For immunoblot detection, the ECL system from NEN was used. cDNAs used for transient transfection were in a cytomegalovirus-based expression plasmid. HA-Mig-6 was fused to an N-terminal HA epitope (Meloche et al., 1992). cDNAs used for viral infection were in pLXSN constructs. Mig-6 was cloned by PCR using the primers 5'-CGGAATTCGCCACCATGTCAACAGCAGGAGTTGCTGC-3' and 5'-CGCGGATCCTCAACACGCAGGAGTTGCTGCTC-3'. The PCR-product was digested with EcoR1 and Xbal and ligated into pcDNA3.1.

### 1.2 Yeast two hybrid screen

All protocols were performed essentially as described in the yeast protocols handbook (Clontech). A LexA-based, gal inducible yeast-two hybrid system was used (Gyuris et al., 1993). The yeast strain EGY 48 (MATa, trp1, ura3, his3, LEU2::pLEXAop6-LEU2), the plasmids pEG202, pSH18-34, pJG4-5 and a human liver library in pJG4-5 were gifts from Sugen Inc. A cDNA encoding human EGFR residues 645-1186 was subcloned into the LexA fusion expressing vector pEG202 by PCR. The yeast reporter strain EGY48 was sequentially transformed by the lithium-acetate method with the bait and library constructs. Positive interactor clones were selected by leucine auxotrophy and galactose-inducible β-galactosidase activity. The library construct was recovered from the positive clones with Escherichia coli strain KC8 (parF::Tn5, hsdr, leuB600, trpC9830, lacD74, strA, galK, hisB436) and retransformed into yeast strain EGY48 carrying a LexA-laminin fusion to eliminate unspecific positive clones. The sequence of the selected clones was used to search nucleotdide databases of the National Center for Biotechnology Information using the BLAST algorithm (Altschul et al., 1997).

### 1.3 Cell transfection, lysis, immunoprecipitation and immunoblotting

Cos-7 cells were transiently transfected using Lipofectamine essentially as described (Daub et al., 1997). 80-90% confluent cells were starved, stimulated as indicated and washed once with phosphate-buffered saline. Cells were lysed for 10 min on ice in buffer containing 50 mM Hepes pH 7.5, 150 mM NaCI, 1% Triton X-100, 1 mM EDTA, 10% glycerol, 10 mM sodium pyrophosphate, 2 mM sodium orthovanadate, 10 mM sodium fluoride, 1 mM phenylmethylsulfonyl fluoride and 10 µl/ml aprotinin. Lysates were pre-cleared by centrifugation at 13 000 r.p.m. for 10 min at 4°C. Supernatants were diluted with an equal volume of HNTG buffer (Seedorf et al., 1994) and subsequently immunoprecipitated using the respective antibodies and 30 µl of protein A-Sepharose for 4 h at 4°C. Precipitates were washed three times with HNTG buffer, suspended in SDS sample buffer, boiled and subjected to gel electrophoresis on 7.5% gels. Following SDS-PAGE, proteins were transferred to a nitrocellulose membrane and immunoblotted.

### 1.4 MAPK-assay

MAPK assays were performed as described previously (Daub et al., 1997). Epitope-tagged HA-Erk2 was immunoprecipitated from lysates obtained from 12-well dishes using 2.5 µg of 12CA5 antibody. Immunoprecipitates were washed three times with HNTG buffer and once with kinase buffer (20 mM Hepes pH 7.5, 10 mM MgCl₂, 1 mM dithiothreitol, 200µM sodium orthovanadate). Subsequently, kinase reactions were performed in 30 µl of kinase buffer supplemented with 0.5 mg/ml MBP, 50 µM ATP and 1 µCi of γ-³²P-ATP for 10 min at room temperature. Reactions were stopped by addition of 30 µl of 2 x SDS sample buffer and subjected to gel electrophoresis on 15% gels. The proteins were transferred to nitrocellulose and the upper part was probed with anti-Erk2 antibody. The labeled MBP in the lower part was quantified using a Phosphorimager (Fuji).

### 1.5 RNA extraction and Northern blot analysis

One 10 cm plate of 80-90% confluent Rat-1 cells was treated with inhibitors and agonists as indicated. Total RNA was isolated using the QIAshredder (QIAGEN) and RNeasy (QIAGEN) kits according to the manufacturers' recommendations. RNA samples (5-10 µg) were fractionated in a 1.4% agarose-formaldehyde gel and transferred to a nitrocellulose membrane by upward capillary transfer. cDNA probes were labeled with α-³²P-ATP using the megaprime labeling system from Amersham Pharmacia Biotech. Hybridization was carried out in formamide solution. Blots were exposed using intensifying screens.

### 1.6 Downregulation of the EGFR

Rat-1 cells stably expressing Mig-6 cDNA or control cells were superinfected with supernatants of GR+E 86 releasing high titer pLXSN-EGFR virus for 4-8 hours in the presence of Polybrene (4 µg/ml; Aldrich). After infection, cells were trypsinized and seeded on 12-well dishes (110'000 cells/well). 12 hours later cells were starved and stimulated with EGFR as indicated. The medium was aspirated and the cells were washed twice with ice-cold DHB (DMEM with 20 mM Hepes pH 7.5 and 0.1% bovine serum albumin (BSA)). Surface-bound ligand was removed by acid stripping at 0°C (200 mM acetic acid pH 2.5, 500 mM NaCI) for 6 min and cells were washed three times with DHB. The number of ligand-binding sites on the cell surface was then determined by incubating cells with ¹²⁵I-EGF at 4°C for at least 2 hours. After washing away the unbound radiolabeled ligand three times with ice-cold DHB the surface bound EGF was removed by acid stripping and the radioactivity was counted with a Beckmann 400 gamma counter.

### 1.7 Focus formation

Ecotopic virus producing cell cultures (GP+E 86) were used to infect subconfluent Rat-1 fibroblasts (10⁵ cells/6-cm dish) for 4 hours in the presence of polybrene (4µg/ml). Equal amounts of retrovirus containing the cDNAs of wildtype EGFR, *v*-fms or the empty vector (pLXSN) (m.o.i. = 0.1-0.25) were used for infection. Cells were cultured in 4% FCS with or without the addition of 10 ng/ml EGF. Medium was changed every other day. After 14 days, cells were fixed with 4% paraformaldehyde and stained with crystal violet (30% methanol, 0.1% crystal violet).

### 2. Results

### 2.1 Identification of Mig-6 as an interaction partner of the EGFR

To identify negative regulating proteins of the EGFR signalling pathway, a yeast two-hybrid screen was performed. The hybrid-bait consisted of the intracellular domain of the human EGFR (amino acid 645-1186) and a galactose inducible, LexA based yeast two-hybrid system (Gyuris et al., 1993) was used. The interaction between the EGFR and Shc, which requires tyrosine phosphorylation of the receptor, was used as control for the activation state of the EGFR in yeast. Shc interaction was detected with the intracellular domain of the wildtype EGFR but not the kinase-inactive K721A mutant (data not shown) which demonstrated that our two-hybrid EGFR construct mimiced an activated, tyrosine phosphorylated receptor. Using a kinase active EGFR construct as bait we screened human liver and heart cDNA libraries. The identification of several known interaction partners in both libraries such as Grb2, Shc and p85 confirmed the validity of our approach. Besides these well characterized proteins, three and two independent clones in the liver and heart libraries encoding different portions of a protein designated Mig-6 were identified respectively. Mig-6 has previously been described but so far not functionally characterized (Wick et al. 1995).

A Mig-6 was first cloned in 1995 by Wick and coworkers (1995) by screening a library of lung fibroblasts that had been stimulated with fetal calf serum for inducing genes. The 462 amino acid sequence exhibits a significant homology of 84% with the rat gene 33 product which consists of 459 amino acids (Chrapkiewicz et al., 1989). Analysis of the amino acid sequence with database and pattern searches revealed several potential serine/threonine phosphorylation sites, a potential Cdc42 and rac interaction binding domain (CRIB) (Burbelo et al., 1995), several SH3 and WW domain-binding motifs and a serine- and proline-rich carboxy terminal domain, that shows within a sequence of 134 amino acids a homology of 71 % with the cytoplasmic tyrosine kinase ACK1 (Manser et al., 1993) but no homology with the related ACK2 as shown in Fig. 1 besides a cytoplasmic localization and regulation of its mRNA during the cell cycle (Wick et al., 1995) no functional information on Mig-6 has been reported so far. Since only the amino acid sequene of the human Mig-6 protein had been published at the beginning of this work and no full-length cDNA had been isolated in our two-hybrid screen, we cloned the rat homologue (genebank RRG33A) from Rat-1 fibroblast cDNA by PCR and used it in the subsequent experiments.

To determine regions in the EGFR sequence that are necessary for binding Mig-6, sequential truncation mutants were used in the two-hybrid system.

The expression of all truncation mutants in yeast was verified by immunoblotting (data not shown) and interactions were tested by monitoring the transcription of the nutritional LEU2 and the LacZ reporter genes. Deletion of the last 192 amino acids from the carboxy-terminus had no effect on the interaction (Figure 2). Further truncation of eleven amino acids abolished binding in the yeast two-hybrid system, thereby defining residues 985-995 carboxy-proximal to the kinase domain of the EGFR as essential for binding Mig-6. Moreover, interaction between Mig-6 and the receptor was detected with the wildtype but not with the kinase impaired mutant K721A suggesting the involvement of a phosphorylated tyrosine residue (Figure 2). Mutation of the only tyrosine codon in the region at position 992, however, did not abolish Mig-6 binding to the kinase active EGFR-bait in the yeast two-hybrid system. This result established that the interaction between the EGFR and Mig-6 requires receptor kinase activity, but excludes Y992 as an essential binding site.

### 2.2 Mig-6 antagonizes EGFR signalling

The in vivo interaction between Mig-6 and the EGFR was verified by performing coimmunoprecipitation experiments. Cos-7 cells were transiently transfected with an amino terminally hemagglutinin (HA) epitope-tagged version of Mig-6 (HA-Mig-6) and immunoprecipitations with an anti-HA antibody were performed. Endogenous EGFR was coprecipitated only in cells that were stimulated with EGF but not unstimulated cells (Figure 3A), a result which was in agreement with our findings in the yeast two-hybrid experiments where no interaction was detected with the kinase dead EGFR mutant K721A.

Since binding of the Mig-6 to the EGFR is dependent on EGF-induced active state of the kinase, we investigated, if Mig-6 is also a substrate of this activity. An Mig-6 cDNA expression plasmid was transfected into Cos-7 cells and after immunoprecipitation with anti-HA antibody and polyacrylamid gel electrophoresis, immunoblot analysis was performed using an anti-phosphotyrosine antibody. No phosphotyrosine signal was detected in either unstimulated or EGF treated cells. Phosphoamino acid analysis (Figure 3B) of HA-Mig-6 from ³²P labeled cells further demonstrated the presence of phosphoserine and phosphothreonine but no phosphotyrosine in either the unstimulated state or upon EGF stimulation for up to 60 minutes. However, treatment of cells with the protein tyrosine phosphatase inhibitor pervanadate in Mig-6 tyrosine phosphorylation as shown by anti-phosphorylation as shown by anti-phosphotyrosine immunoblotting of the anti HA-tag immunoprecipitated HA-Mig-6 protein (data not shown). Taken together, these results allow the conclusion that Mig-6 is phosphorylated on serine and threonine residues and that the steady state level of tyrosine phosphorylation level of this protein is either undectably low i.e. tightly regulated by a PTP and/or it is substrate of a protein tyrosine kinase other than the EGFR.

The involvement of Mig-6 in EGFR-signalling was further investigated using Cos-7 cells transiently expressing Mig-6. First, we examined tyrosine phosphorylation of the EGFR in transfected and non transfected cells upon activation with EGF. After 2 min of stimulation, EGFR tyrosine phosphorylation was induced in both cell cultures, but no significant difference in phosphotyrosine content was detected. However, after a stimulation time of 10 min or longer, tyrosine phosphorylation of the EGFR was dramatically reduced in cells expressing Mig-6 relative to control cells (Figure 4A). These findings led us to question, if downstream signalling of the EGFR is also negatively influenced by expression of Mig-6. We therefore investigated the activation of mitogen-activated protein kinase (MAPK) Erk2 using a transiently expressed hemagglutinin (HA) epitope-tagged form of Erk2 (HA-Erk2) and measured the incorporation of ³²P in myelin basic protein (MBP). Expression of Mig-6 did reduce activation of Erk2 by 65% upon direct EGFR stimulation with EGF and by 50% and 62% upon EGFR transactivation by the GPCR agonists LPA or thrombin, respectively (Figure 4B). In contrast, Mig-6 had no influence on MAPK activation upon PDGF stimulation, whereas MAPK activity was even increased by 30% in Mig-6 expressing cells upon FGF stimulation (Figure 4B).

### 2.3 Mig-6 induced EGFR downregulation

The antagonistic effects of Mig-6 in EGFR-signalling could be explained by direct inhibition of EGFR kinase activity, by interference with the transphosphorylation reaction within the receptor dimer, by activation of an EGFR PTR or by an involvement of Mig-6 in the process of downregulation. Since internalization of RTKs upon stimulation is considered to be a major attenuation mechanism (Sorkin and Waters, 1993a), we analyzed the effect of Mig-6 on the fraction of surface-exposed EGF-binding sites in Rat-1 fibroblasts. Rat-1 cells stably expressing Mig-6 (clone 19 and 23) or control cells (mock) were generated by infection with a retrovirus containing the cDNA of Mig-6 or the empty vector as described in Materials and Methods. After stimulation, the cells were stripped to remove the surface-bound EGF and incubated with ¹²⁵I-EGF. After washing the surface bound radioactivity was counted. Within 5 min of EGF stimulation, only 25% of the surface EGF-binding sites disappeared on the control cells. In contrast, in cells that stably expressed Mig-6 about 45%-55% of the surface binding sites were downregulated within 5 min of stimulation (Figure 5). After 20 min of stimulation 20% of the surface receptors were still present on control cells compared to only 10% on the Mig-6 expressing cells. Interestingly, analysis of Mig-6 transfected and control cells indicated that the EGFR half life of the endogenous EGFR was extended by Mig-6 overexpression from 11 to 14 hours (data not shown) suggesting no involvement in EGFR degradation.

### 2.4 EGF induces Mig-6 mRNA expression

Since Mig-6 is involved in EGF-signal regulation and both human and rat cDNA were originally identified by screening libraries of stimulated cells (Chrapkiewicz et al., 1989; Lee et al., 1985; Wick et al., 1995), we investigated, if EGF induces Mig-6 mRNA production in Rat-1 fibroblasts. Northern blot analysis confirmed that Mig-6 mRNA is expressed only at low levels in starved cells (Figure 6A). Upon stimulation with EGF, Mig-6 mRNA levels are elevated within 30 min followed by a decline after one hour, whereas upon stimulation with FCS the highest expression is reached after one hour (Figure 6A). Similarly, the GPCR agonists LPA and thrombin, which transactivate the EGFR signal (Daub et al., 1996), also cause induction of Mig-6 mRNA expression (Figure 6B). To confirm that induction upon EGF, LPA, thrombin and FCS stimulation requires EGFR activity, we employed the EGFR-specific inhibitor AG1478. As shown in Figure 6B, pre-incubation of cells with AG1478 markedly reduced Mig-6 mRNA expression demonstrating the the EGFR signal was critical for Mig-6 gene transcription in response to EGF, FCS or the GPCR agonists LPA and thrombin. Stimulation with FGF did not induce expression of Mig-6 mRNA, whereas PDGF treatment induced a marginal effect (Figure 6C). To obtain information regarding the signalling pathways involved in Mig-6 gene activation we used pathway-selective inhibitors on EGF stimulated Rat-1 cells followed by RNA extraction and Northern blot analysis. No effect was seen with the p38 MAPK inhibitors SB203580 and SB202190 or with the PI3K inhibitors Ly29902 and wortmannin (data not shown). In contrast, the MAP kinase kinase (MEK) inhibitor PD98995 completely inhibited the induction of Mig-6 mRNA upon EGF stimulation (Figure 6D) demonstrating that activation of the ERK/MAPK pathway is necessary for Mig-6 transcription upon EGF stimulation.

### 2.5 Mig-6 inhibits EGF-induced cell transformation

EGFR gene amplification and overexpression has been demonstrated in a variety of primary tumor types (Hesketh, 1997) and in NIH3T3 experimental models the transforming potential of the EGFR had been established (Di Fiore et al., 1987; Riedel et al., 1988). Since Mig-6 antagonized EGFR autophosphorylation and -signalling, we asked whether it is able to inhibit EGFR-induced cell transformation. We therefore performed focus formation assays with Rat-1 fibroblasts that express Mig-6 (clones 19, 23) or with control cells. The fibroblasts were superinfected with recombinant retrovirus containing EGFR coding sequence, *v*-fms or control virus. Figure 7 shows that expression of Mig-6 completely inhibited EGFR dependent focus formation in cell monolayers when compared to control cells. The viral oncogene *v*-fms transformed Rat-1 cells as well, but focus formation induced by *v*-fms was not inhibited by Mig-6 expression. Based on these findings we conclude that Mig-6 is a selective negative control element involved in the regulation of cell responses to EGFR ligands.

### 3. Discussion

We have identified Mig-6 as a novel binding partner of the EGFR and characterized its antagonistic activity on the EGFR-signal. The protein contains several possible serine/threonine phosphorylation sites, a number of minimal SH3 and WW domain-binding sequences and a CRIB motif. Furthermore, within the carboxy-terminal domain, Mig-6 exhibits a high degree of sequence homology with ACK1 which belongs together with ACK2 to a family of cytoplasmic tyrosine kinases. Both family members contain an SH3 domain, a CRIB motif and a proline rich carboxy-terminal domain. Interestingly, ACK2 does not share sequence homology with Mig-6 because of a 213 amino acid homology gap in the respective portion of the molecule. Since ACK2 mediates cell adhesion signals whereas Mig-6 and ACK1 are both involved in EGFR-signalling (Yang et al., 1999; Satoh et al., 1996), it is tempting to speculate that this domain is relevant for their involvement in EGFR signal transduction. Furthermore, Mig-6 and ACK1 both contain a CRIB motif which is comprised of a sequence that binds Rac and Cdc42 (Baulida et al., 1996) both of which are members of the Rho family of small GTPases. These signal transducing proteins are involved in different signal-transduction pathways (Chou and Blenis, 1996; Coso et al., 1995; Minden et al., 1995) as well as in the rearrangement of the cytoskeleton (Nobes and Hall, 1995; Ridley et al., 1992). Binding of Cdc42 or Rac to CRIB motifs is GTP-dependent which suggests that CRIB motif containing proteins are targets of activated Cdc42 or Rac. A number of CRIB motif containing kinases like ACK2 or Pak are activated upon binding of Cdc42 or Rac (Manser et al., 1994; Yang and Cerione, 1997). An interaction between Cdc42 or Rac with Mig-6 could induce a conformational change which either results in the creation of binding sites for additional partners or prevents other proteins from interacting. Thus, Mig-6 could act as an adapter molecule whose interaction with other proteins is regulated by binding of either Cdc42 or Rac.

Such as possible function is supported by the presence of a number of different SH3 and WW domain-binding motifs in the Mig-6 sequence enable it to interact with other proteins. Thus, Mig-6 and ACK1 are potentially both involved in signal transduction by RTKs and small GTPases and could function as point of convergence and integration of different signal transduction pathways.

The adapter protein Shc is a well known interaction partner of the EGF to which it binds directly in a phosphotyrosine binding (PTB) domain- and phosphotyrosine-dependent manner. In our yeast two-hybrid experiments, Shc binds the intracellular domain of the wildtype EGFR, but not that of a kinase-inactive receptor mutant. From this result we concluded that the wildtype EGFR-bait mimics the activated and autophosphorylated state of the receptor. Similarly, the interaction between Mig-6 and the EGFR is dependent on the tyrosine kinase activity of the receptor in the yeast two-hybrid system. The site of interaction was mapped to amino acids 985-995 in the carboxy terminal regulatory domain of the EGFR (Figure 2). While these observations may suggest that in spite of the absence of an SH2 or PTB domain tyrosine 992 within this ten amino acid sequence may upon phosphorylation (Walton et al., 1990) form a binding site for Mig-6, we found that replacement of this residue with phenylalanine did not abolish binding to the kinase-active EGFR. The EGFR-Mig-6 interaction is therefore dependent on kinase activity but not directly on tyrosine phosphorylation, an observation that suggests the formation of a Mig-6 binding site by a conformational change of the receptor upon its activation (Cadena et al., 1994). Interestingly, the 985-995 EGFR sequence is part of a previously identified domain that is involved in calcium signalling and receptor internalization. Moreover, this as CALN domain designated region displays a high content of negatively charged amino acids which strongly supports a function as protein interaction site on the surface of the kinase domain (Chen et al., 1989).

The early observations of Gill and coworkers (Chen et al., 1989) provide now support for our investigation of Mig-6 function. Overexpression of Mig-6 accelerated the dephosphorylation rate of the EGFR and inhibited the extent of MAP kinase activation upon stimulation with EGF, but not PDGF or FGF. In addition, Mig-6 reduced MAP kinase activation upon stimulation with the GPCR agonists LPA and thrombin which require EGFR kinase activity for the activation of the Ras-MAP kinase pathway (Daub et al., 1997). These results demonstrate that Mig-6 acts as a potent and selective antagonist of EGFR function.

Signals of RTKs are not only defined by qualitative parameters but also by the duration of their activation (Traverse et al., 1992). Mig-6 might represent an important element in a mechanism that defines the cellular response to EGFR ligand stimulation. A negative regulatory effect may either be due to enhanced dephosphorylation of the receptor or by its accelerated downregulation, a process that includes internalization and recycling or degradation. Since the overexpression of Mig-6 reduced the number of binding sites for EGF on the surface of cells, it seems likely that Mig-6 is involved in receptor downregulation. In this scenario Mig-6 could either recruit molecules involved in receptor endocytosis and therefore influence the kinetics of internalization or alternatively, the EGFR could be delivered more rapidly to a compartment where it is efficiently dephosphorylated and trapped in a "non-recycling" state after its internalization. Sorting of the receptor to the degradation pathway by Mig-6 can be excluded since expression of Mig-6 did not decrease the half life of the receptor. Since Mig-6 is involved in dephosphorylation as well as downregulation, the targeting of a phosphatase to the receptor could be an additional function of Mig-6. Furthermore, the small GTPase Rac has been described to regulate clathrin-coated vesicle formation and endocytosis. (Lamaze et al., 1996), which raises the interesting possibility of an interaction between Mig-6 and Rac and therefore an involvement of Rac in the downregulation of the EGFR.

Mig-6 does not only negatively regulate EGF-signalling, its transcription is also induced upon activation of the EGFR. In Rat-1 fibroblasts, Mig-6 mRNA expression is enhanced upon stimulation with FCS, EGF, LPA and thrombin, but not upon FGF and only marginaly upon PDGF stimulation. To induce Mig-6 transcription upon stimulation with FCS, LPA and thrombin the kinase activity is required. Thus Mig-6 appears to be part of a negative feedback mechanism that regulates the duration of the EGFR signal. Such negative feedback loops have been demonstrated in different major pathways, such as cytokine- (Endo et al., 1997; Naka et al., 1997; Starr et al., 1997), TGF-β- (Stroschein et al., 1999) or RTK-signalling (Seedorf et al., 1995; Ghiglione et al., 1999). In Drosophila oogenesis, the spatial activity of the EGFR is regulated by the two proteins kek1 and Argos in separate negative feedback loops. In this case the secreted protein Argos interacts in a paracrine manner directly with the extracellular domain of the EGFR (Freeman, 1996), whereas an interaction with the extracellular as well as the transmembrane domain of the EGFR is required for kek1 to inhibit the receptor (Ghiglione et al., 1999). In both cases the inhibitory proteins interact with the extracellular domain of the EGFR and might therefore inhibit the binding of the ligand to its receptor. Mig-6 on the contrary, is localized to the cytoplasm and interacts with the carboxy-terminal regulatory domain of the receptor which makes a direct interference with the binding of EGF unlikely. Whereas Argos and kek1 have only been described during Drosophila oogenesis, we found that Mig-6 is expressed in terminally differentiated tissues such as liver, kidney, lung and brain (data not shown). Thus, negative feedback regulation occurs not only during development and represents a critical mechanism for EGFR signal regulation and definition.

We have shown that enhanced expression of Mig-6 suppresses focus formation induced by EGFR overexpression but not by other oncogenes such as *v*-fms. This shows that Mig-6 specifically controls EGFR-signalling and impairment of this attenuation mechanism may lead to increased cell proliferation and tumor formation. Since the first demonstration of a connection between the EGFR and the *v*-erbB oncogene (Downward et al., 1984) and demonstration of EGFR gene amplification in A431 cells (Ullrich et al., 1984) the importance of this RTK as a major element in the promotion of tumor growth and invasion has been suggested for a variety of cancers. Bescause oncogenic dysregulation of RTK signals always involves the incapacitation of negatively regulating tumor suppressor gene products our findings regarding the downregulating function of Mig-6 provide new insights which have broad diagnostic and therapeutic significance.

### References

Altschul, S.F. Madden, T.L., Schäffer, A.A., Zhang, J., Zhang, Z., Miller, W. and Lipman, D.J. (1997). Gapped BLAST and PSI-BLAST: a new generation of protein database search programs. Nucleic Acids Res., 25, 3389-3402.

Baulida, J., Kraus, M.H., Alimandi, M., Fiore, P.P.D. and Carpenter, G. (1996). All ErbB receptors other than the epidermal growth factor receptor are encytosis impaired. J. Biol. Chem., 271, 5251-5257.

Burbelo, P.D., Drechsel, D. and Hall, A. (1995). A conserved binding motif defines numerous candidate target proteins for both Cdc42 and Rac GTPases. J. Biol. Chem., 270, 29071-29074.

Cadena, D., Chan, C.L. and Gill, G.N. (1994). The intracellular tyrosine kinase domain of the epidermal growth factor receptor undergoes a conformational change upon autophosphorylation. J. Biol. Chem., 269, 260-265.

Chang, C.-P., Lazar, C.S., Walsh, B.J., Komuro, M., Collawn, J.F., Kuhn, L.A., Trainer, J.A., Trowbridge, I.S., Farquhar, M.G., Rosenfeld, M.G., Wiley, H.S. and Gill, G.N. (1993). Ligand-induced internalization of the epidermal growth factor receptor is mediated by multiple endocytic codes analogous to the tyrosine motif found in constitutively internalized receptor. J. Biol. Chem., 268, 19312-19320.

Chen, W.S., Lazar, C.S., Lund, K.A., Welsh, J.B., Chang, C.P., Walton, G.M., Der, C.J., Wiley, H.S., Gill, G.N. and Rosenfeld, M.G. (1989). Functional independence of the epidermal growth factor receptor from a domain required for ligand induced internalization and calcium regulation. Cell, 59, 33-43.

Chou, M.M. and Blenis, J. (1996). The 70 kDa S6 kinase complexes with and is activated by the Rho family G proteins Cdc42 and Rac1. Cell, 85, 573-583.

Chrapkiewicz, N.B., Davis, C.M., Chu, D.T.-W., Caldwell, C.M. and Granner, D.K. (1989). Rat gene 33: analysis of its structure, messenger RNA and basal promoter activity, Nucleic Acids Res., 17, 6651-6667.

Coso, O.A., Chiariello, M., Yu, J.-C., Teramoto, H., Crespo, P., Xu, N., Miki, T. and Gutkind, J.S. (1995). The small GTP-binding proteins Rac1 and Cdc42 regulate the activity of the JNK/SAPK signalling pathway. Cell, 81, 1137-1146.

Countaway, J.L., Nairns, A.C. and Davis, R.J. (1992). Mechanism of desensitization of the epidermal growth factor receptor protein-tyrosine kinase. J. Biol. Chem., 267, 1129-1140.

Daub, H., Wallasch, C., Lankenau, A., Herrlich, A. and Ullrich, A. (1997). Signal characteristics of G protein-transactivated EGF receptor. EMBO J., 16, 101-113.

Daub, H., Weiss, F.U., Wallasch, C. and Ullrich, A. (1996). Role of transactivation of the EGF receptor in signalling by G-protein-coupled receptors. Nature, 379, 557-560.

Di Fiore, P.P., Pierce, J.H., Fleming, T.P., Hazan, R., Ullrich, A., King, C.R, and Schlessinger, J. (1987). Overexpression of the human EGF receptor confers an EGF-dependent transformed phenotype to NIH 3T3 cells. Cell, 51, 1063-1070.

Downward, J., Yarden, A., Mayes, E., Scrace, G., Totty, N., Stockwell, P., Ullrich, A., Schlessinger, J. and Waterfield, M.D. (1984). Close similarity of epidermal growth factor receptor and v-erb-B oncogene protein sequences. Nature, 307, 521-527.

Ekstrand, A.J., Sugawa, N., James, C.D. and Collins, V.P. (1992). Amplified and rearraged epidermal growth factor receptor genes in human glioblastimas reveal deletions of sequences encoding portions of the N-and/or C-terminal tails. Proc. Natl. Acad. Sci. USA, 89, 4309-4013.

Endo, T.A., Masuhara, M., Yokouchi, M., Suzuki, R., Sakamoto, H., Mitsui, K., Matsumoto, A., Tanimura, S., Ohtsubo, M., Misawa, H., Miyazaki, T., Leonor, N., Taniguchi, T., Fujita, T., Kanakura, Y., Komiya, S. and Yoshimura, A. (1997). A new protein containing an SH2 domain that inhibits JAK kinases. Nature, 387, 921-924.

Fiorentino L., et al. (2000). Inhibition of ErbB-2 Mitogenic and Transforming Activity by RALT, a Mitogen-Induced Signal Transducer Which Binds to the ErbB-2 Kinase Domain. Mol. Cell. Biol., 20, 7735-7750.

Freeman, M. (1996). Reiterative use of the EGF receptor triggers differentiation of all cell types in the Drosophilia eye. Cell, 87, 651-660.

Futter, C.E., Felder, S., Schlessinger, J., Ullrich, A. and Hopkins, C.R. (1993). Annexin I is phosphorylated in the multivesicular body during the processing of the epidermal growth factor receptor. J. Cell Biol., 120, 77-83.

Ghiglione, C., Carrawaylll, K.L., Amundadottir, L.T., Boswell, R.E., Perrimon, N. and Duffy, J.B. (1999). The transmembrane molecule Kekkon 1 acts in a feedback loop to negatively regulate the activity of the Drosophila EGF receptor during oogensis. Cell, 96, 847-856.

Gyuris, J., Golemis, E., Chertkov, H. and Brent, R. (1993). Cdi1, a human Gl and S phase protein phosphatase that associates with Cdk2. Cell, 75, 791-803.

Hackel, P.O., Zwick, E., Prenzel, N. and Ullrich, A. (1999). Epidermal growth factor receptors: critical mediators for multiple receptor pathways. Curr. Opin. Cell Biol., 11, 184-189.

Heisermann, G.J. and Gill., G.N. (1988). Epidermal growth factor receptor threonine and serine residues phosphorylated in vivo. J. Biol. Chem., 263, 13152-13158.

Hesketh, R. (1997). The oncogene and tumour suppressor gene Facts Book. Academic Press, San Diego, USA.

Honegger, A.M., Dull, T.J., Felder, S., vanObberghen, E., Bellot, F., Szapary, D., Schmidt, A., Ullrich, A. and Schlessinger, J. (1987). Point mutation of the ATP binding site of EGF receptor abolishes protein-tyrosine kinase activity and alters cellular routing. Cell, 51, 199-209.

Horak, E., Smith, K., Bromley, L., LeJeune, S., Greenall, M., Lane, D. and Harris, A.L. (1991). Mutant p53, EGF receptor and c-erbB-2 expression in human breast cancer. Oncogene, 6, 2277-2284.

Joazeiro, C.A.P., Wing, S.S., Huang, H.-K., Leverson, J.D., Hunter, T. and Liu, Y.-C. (1999). The tyrosine kinase negative regulator c-Cbl as a RING-type E2-dependent ubiquitin-protein ligase. Science, 286, 309-312.

Joly, M., Kazlauskas, A. and Corvera, S. (1995). Phosphatidylinositol 3-kinase activity is required at a postendocytic step in platelet-derived growth factor receptor trafficking. J. Biol. Chem., 270, 13225-13230.

Kharitonenkov, A., Chen., Z., Sures, I., Wang, H., Schilling, J. and Ullrich, A. (1997). A family of proteins that inhibit singalling through tyrosine kinase receptors. Nature, 386, 181-186.

Kornilova, E., Sorkina, T., Beguinot, L. and Sorkin, A. (1996). Lysosomal targeting of epidermal growth factor receptors via a kinase-dependent pathway is mediated by the receptor carboxy-terminal residues 1022-1123. J. Biol. Chem., 271, 30340-30346.

Kuppuswamy, D., Dalton, M. and Pike, L.J. (1993). Serine 1002 is a site of in vivo and in vitro phosphorylation of the epidermal growth factor receptor. J. Biol. Chem., 268, 19134-19142.

Kurten, R.C., Cadena, D.L. and Gill, G.N. (1996). Enhanced degradation of EGF receptors by a sorting Nexin, SNX1. Science, 272, 1008-1010.

Lamaze, C., Chuang, T.-H., Terlecky, L.J., Bikoch, G.M. and Schmid, S.L. (1996). Regulation of receptor-mediated endocytosis by Rho and Rac. Nature, 382, 177-179.

Lamaze, C. and Schmid, S.L. (1995). Recruitment of epidermal growth factor into coated pits requires their activated tyrosine kinase. J. Cell Biol., 129, 47-54.

Lee, K., Isham, K.R., Stringfellow, L., Rothrock, R. and Kenney, F.T. (1995). Molecular cloning of cDNAs as cognate to genes sensitive to hormonal control in rat liver. J. Biol. Chem., 260, 16433-16438.

Levkowitz, G. Watermann, H., Zamir, E., Kam, Z., Oved, S., Langdon, W.Y., Beguinot, L., Geiger, B. and Yarden, Y. (1998). c-Cbl/Sli-1 regulates endocyctic sorting and ubiquitination of the epidermal growth factor receptor. Genes & Development, 12, 3663-3674.

Liu, F. and Chernoff, J. (1997). Protein tyrosine phosphatase 1B interacts with and is tyrosine phosphorylated by the epidermal growth factor receptor. Biochem J, 327, 139-145.

Manser, E., Leung, T., Salihuddin, H., Tan, L. and Lim, L. (1993). A non-receptor tyrosine kinase that inhibits the GTPase activity of p21Cdc42. Nature, 363, 364-367.

Manser, E., Leung, T., Salihuddin, H., Zhao, Z.S. and Lim, L. (1994). A brain serine/threonine protein kinase activated by Cdc42 and Rac1. Nature, 367, 40-46.

Meloche, S., Pages, G. and Poussegur, J. (1992). Functional expression and growth factor activation of an epitope-tagged p44 mitogen-activated protein kinase p44^{mapk}. Mol. Biol. Cell, 3, 63-71.

Minden, A., Lin, A., Claret, F.-X., Abo, A. and Karin, M. (1995). Selective Activation of the JNK signalling cascade and c-Jun transcriptional activity by the small GTPases Rac and Cdc42Hs. Cell, 81, 1147-1157.

Morrison, P., Saltiel, A.R. and Rosner, M.R. (1996). Role of mitogen-activated protein kinase kinase in regulation of the epidermal growth receptor by protein kinase. C. J. Biol. Chem., 271, 12891-12896.

Naka, T., Narazaki, M., Hirata, M., Matsumoto, T., Minamoto, S., Aono, A., Nishimoto, N., Kajita, T., Taga, T., Yoshizaki, K., Akira, S. and Kishimoto, T. (1997). Structure and function of a new STAT-induced STAT inhibitor. Nature, 387, 924-928.

Nesterov, A., Wiley, H.S. and Gill, G.N. (1995). Ligand-induced endocytosis of epidermal growth factor receptors that are defective in binding adaptor proteins. Proc. Natl. Acad. Sci. USA, 92, 8719-8723.

Nobes, C.D. and Hall, A. (1995). Rho, rac, cdc42 GTPases regulate the assembly of multimolecular focal complexes associated with actin stress fibers, lamilipodia and filopodia. Cell, 81, 53-62.

Proctor, A.J., Coombs, L.M., Cairns, J.P. and Knowles, M.A. (1991). Amplification at chromosome 11q13 in transitional cell tumours of the bladder. Oncogene, 6, 789-795.

QujanaoJr., V.J. and Sheffield, L.G. (1998). Prolactin decreases epidermal growth factor receptor kinase activity via a phosphorylation-dependent mechanism. J. Biol. Chem., 273, 1200-1207.

Radinsky, R., Risin, S., Fan, D., Dong, Z., Bielenberg, D., Bucana, C.D. and Fidler, I.J. (1995). Level and function of epidermal growth factor predict the metastatic potential of human colon carcinoma cells. Clin. Cancer Res., 1, 19-31.

Ridley, A.J., Paterson, H.F., Johnston, C.L. Diekmann, D. and Hall, A. (1992). The small GTP-binding protein rac regulates growth factor-induced membrane ruffling. Cell, 70, 401-410.

Riedel, H., Massoglia, R., Schlessinger, J. and Ullrich, A. (1988). Ligand activation of overexpressed epidermal growth factor receptors transform NIH 3T3 mouse fibroblasts. Proc. Natl. Acad. Sci. USA, 85, 1477-1481.

Riese II, D.J. and Stern, D.F. (1998). Specificity within the EGF family/ErbB receptor family signalling network. BioEssay, 20, 41-48.

Satoh, T., Kato, J., Nishida, K. and Kaziro, Y. (1996). Tyrosine phosphorylation of ACK in response to temperature shift-down, hyperosmotic shock and epidermal growth factor stimulation. FEBS Lett., 386, 230-234.

Seedorf, K., Kostka, G., Lammers, R., Bashkin, P., Daly, R., Burgess, W.H., vanderBliek, A.M., Schlessinger, J. and Ullrich, A. (1994). Dynamin binds to SH3 domains of phospholipase Cy and GRB2. J. Biol. Chem., 269, 16009-16014.

Seedorf, K., Shearman, M. and Ullrich, A. (1995). Rapid and long-term effects of protein kinase C on receptor tyrosine kinase phosphorylation and degradation. J. Biol. Chem., 270, 18953-18960.

Sorkin, A. and Carpenter, G. (1993b). Interaction of activated EGF receptors with coated pit adaptins. Science, 261, 612-615.

Sorkin, A., Helin, K., Waters, C.M., Carpenter, G. and Beguinot, L. (1992). Multiple autophosphorylation sites of the epidermal growth factor receptor are essential for receptor kinase activity and internalization. J. Biol. Chem., 267, 8672-8678.

Sorkin, A. and Waters, C.M. (1993a). Endocytosis of growth factor receptors. BioEssays, 15, 375-382.

Starr, R., Wilson, T., Viney, E.M., Murray, L.J.L., Rayner, J.R., Jenkins, B.J., Gonda, T.J., Alexander, W.S., Metcalf, D., Nicola, N.A. and Hilton, D.J. (1997). A family of cytokine-inducible inhibitors of signalling. Nature, 387, 917-921.

Stroschein, S.L., Wang, W., Zhou, S., Zhou, Q. and Luo, K. (1999). Negative feedback regulation of TGF-β signalling by the SnoN oncoprotein. Science, 286, 771-774.

Theroux, S., Taglienti-Sian, C., Nair, N., Countaway, J.L., Robinson, H.L. and Davis, R.J. (1992a). Increased oncogenic potential of ErbB is associated with the loss of a COOH-terminal domain serine phosphorylation site. J. Biol. Chem., 267, 7967-7970.

Theroux, S.J., Latour, D.A., Stanley, K., Raden, D.L. and Davis, R.J. (1992b). Signal transduction by the epidermal growth factor receptor is attenuated by a COOH-terminal domain serine phosphorylation site. J. Biol. Chem., 267, 16620-16626.

Traverse, S., Gomez, N., Paterson, H., Marshall, C. and Cohen, P. (1992). Sustained activation of the mitogen-activated protein (MAP) kinase cascade may be required for differentiation of PC12 cells. Comparison of the effects of nerve growth factor and epidermal growth factor. Biochem. J., 288, 351-355.

Ullrich, A., Coussens, L., Hayflick, J.S., Dull, T.J., Gray, A., Tam. A.W., Lee, J., Yarden, Y., Libermann, T.A., Schlessinger, J., Downward, J., Mayers, E., Whittle, N., Waterfield, M.D. and Seeburg, P.H. (1984). Human epidermal growth factor receptor cDNA sequence and aberrant expression of the amplified gene in A431 epidermoid carcinoma cells. Nature, 309, 418-425.

Walton, G.M., Chen, W.S., Rosenfeld, M.G. and Gill, G.N. (1990). Analysis of deletions of the carboxy terminus of the epidermal growth factor receptor reveals self-phosphorylation at tyrosine 992 and enhanced in vivo tyrosine phosphorylation of cell substrates. J. Biol. Chem., 265, 1750-1754.

Wang, Z. and Moran, M.F. (1996). Requirement for the adaptor protein GRB2 in EGF receptor endocytosis. Science, 272, 1935-1938.

Watermann, H., Levkowitz, G., Alroy, I. and Yarden, Y. (1999). The RING finger of c-Cbl mediates desensitization of the epidermal growth factor receptor. J. Biol. Chem., 274, 22151-22154.

Wells, A., Welsh, J.B., Lazar, C.S., Wiley, H.S., Gill, G.N. and Rosenfeld, M.G. (1990). Ligand-induced transformation by a noninternalizing epidermal growth factor receptor. Science, 247, 962-964.

Wick, M., Bürger, C., Funk, M. and Müller, R. (1995). Identification of a novel mitogen-inducible gene (mig-6): regulation during G₁ progression and differentiation. Exp. Cell Res., 219, 527-535.

Yang, W. and Cerione, R.A. (1997). Cloning and characterization of a novel Cdc42-associated tyrosine kinase, ACK-2 from bovine brain. J. Biol. Chem., 272, 24819-24824.

Yang, W., Lin, Y., Guan, J.L. and Cerione, R.A. (1999). Activation of the Cdc42-associated tyrosine kinase-2 (ACK-2) by cell adhesion via integrin ß1. J. Biol. Chem., 274, 8524-8530.

Yoon, C.H., Lee, J., Jongeward, G.D. and Sternberg, P.W. (1995). Similarity of sli-1, a regulator of vulval development in C. elegans, to the mammalian proto-oncogene c-cbl. Science, 269, 1102-1105.

Zwick, E., Hackel, P.O., Prenzel, N. and Ullrich, A. (1999). EGF receptors as central regulators of heterologous signalling systems, Trends Pharmacol. Sci., 20, 408-412.

## Claims

1. Use of a Mig-6 protein for the manufacture of an agent for the modulation of Epidermal Growth-Factor Receptor (EGFR) activity.

2. The use of claim 1 wherein the Mig-6 protein is a mammalian Mig-6 protein or a recombinant derivative or variant thereof.

3. The use of claim 1 or 2 wherein the Mig-6 protein comprises
(a) the amino acid sequence as shown in Genbank Accession No. NM_018948 or
(b) an amino acid sequence having an identity of at least 80% thereto.

4. Use of a nucleic acid encoding a Mig-6 protein or a nucleic acid complementary thereto for the manufacture of an agent for the modulation of EGFR activity.

5. The use of claim 4 wherein the nucleic acid encodes a mammalian Mig-6 protein or a recombinant derivative or variant thereof.

6. The use of claim 4 or 5 wherein the nucleic acid comprises:
(a) a nucleic acid sequence as shown in Genbank Accession No. NM_018948 or complementary thereto,
(b) a nucleic acid sequence corresponding to the sequence of (a) within the scope of degeneracy of the genetic code or
(c) a nucleic acid sequence hybridizing under stringent conditions with the sequence of (a) and/or (b).

7. The use of any one of the preceding claims wherein the modulcation of EGFR activity comprises an inhibition of EGFR activity.

8. The use of claim 7 wherein said inhibition of EGFR activity comprises an inhibition of the mitogen-activated protein kinase pathway.

9. The use of claim 7 or 8 wherein said inhibition of EGFR activity comprises an inhibition of Erk2 activity.

10. The use of any one of the preceding claims for the manufacture of an agent for the diagnosis, prevention or treatment of an EGFR overexpression associated disorder.

11. The use of claim 10 wherein said disorder is a hyperproliferative disease.

12. The use of claim 10 or 11 wherein said disorder is selected from inflammatory processes and tumors.

13. Use of a Mig-6 protein as a target for the modulation of EGFR activity.

14. Use of a nucleic acid encoding a Mig-6 protein or a nucleic acid complementary thereto as a target for the modulation of EGFR activity.

15. The use of claim 13 or 14 comprising enhancing the amount and/or activity of a Mig-6 protein.

16. The use of claim 15 comprising an enhanced expression of Mig-6 in a target cell.

17. The use of claim 15 comprising the introduction of a Mig-6 protein activator to a target cell.

18. A composition comprising as an active agent a Mig-6 protein together with pharmaceutically acceptable carriers or diluents.

19. A composition comprising as an active agent a Mig-6 nucleic acid or a nucleic acid complementary thereto together with pharmaceutically acceptable carriers or diluents.

20. The composition of claim 18 or 19 for the use in diagnostic or medical applications.

21. The composition of any one of claims 18-20 for the use in tumor diagnosis, prevention or therapy.

22. A method of identifying novel modulators of EGFR activity comprising screening for substances having equivalent biological activity as Mig-6.

23. The method of claim 22 wherein said biological activity comprises binding to EGFR in a region between amino acids 985 and 995.

24. The method of claim 23 wherein said binding is independent of tyrosine 992.

25. The method of claim 22 wherein said biological activity comprises a selective inhibition of the mitogen-activated protein kinase pathway in response to EGF.
